Europäisches Patentamt

European Patent Office

Office européen des brevets

(19)

(11) Publication number: **0 041 174**
**B1**

## EUROPEAN PATENT SPECIFICATION

(12)

(45) Date of publication of patent specification: **11.09.85**

(21) Application number: **81103792.8**

(22) Date of filing: **18.05.81**

(51) Int. Cl.⁴: **C 07 K 15/06, A 61 K 35/14,**
**A 61 K 37/02, B 01 D 15/00**

(54) Blood coagulation promoting product and process of preparing same.

(30) Priority: **27.05.80 US 153396**

(43) Date of publication of application:
**09.12.81 Bulletin 81/49**

(45) Publication of the grant of the patent:
**11.09.85 Bulletin 85/37**

(84) Designated Contracting States:
**AT BE DE FR GB IT SE**

(56) References cited:
**DE-A-2 645 466**
**DE-A-2 734 821**
**DE-A-2 902 158**

(73) Proprietor: **MILES LABORATORIES, INC.**
**1127 Myrtle Street**
**Elkhart Indiana 46514 (US)**

(72) Inventor: **Gautam, Mitra**
**40 Cowper Avenue**
**Kensington California 94707 (US)**

(74) Representative: **Senftl, Hannes, Dr. et al**
**c/o Bayer AG Konzernverwaltung RP**
**Patentabteilung**
**D-5090 Leverkusen Bayerwerk (DE)**

Courier Press, Leamington Spa, England.

# 0 041 174

**Description**

Background of the invention
Field of the invention

This invention relates to and has among its objects the provision of novel blood coagulation components and novel methods of making them. Further objects of the invention will be evident from the following description.

Description of the prior art

There are estimated to be 100,000 cases of congential hemophilia in the United States. Of these, approximately 20,000 are cases of hemophilia B, the blood of such patients being either totally devoid of plasma thromboplastin component or seriously deficient in plasma thromboplastin component. The disease therefore exists in varying degrees of severity, requiring therapy anywhere from every week up to once or twice a year. The completely deficient cases require replacement therapy once every week; the partially deficient cases require therapy only when bleeding episodes occur, which may be as seldom as once a year. The bleeding episodes in congenital, partially-deficient cases are generally caused by a temporarily acquired susceptibility rather than by injury alone. Intravenous injection of a sufficiently large amount of fresh plasma, or an equivalent amount of fresh blood, temporarily corrects the defect of a deficient subject. The beneficial effect often lasts for two or three weeks, although the coagulation defect as measured by in-vitro tests on the patient's blood appears improved for only two or three days. Such therapy with fresh plasma or fresh blood is effective but it has several serious drawbacks: (1) it requires ready availability of a large amount of fresh plasma; (2) requires hospitalization for the administration of the plasma; (3) a great many of the patients become sensitized to repeated blood or plasma infusions and ultimately encounter fatal transfusion reactions; (4) at best plasma can only partially alleviate the deficiency; and (5) prolonged treatment or surgery is not possible because the large amounts of blood or plasma which are required will cause acute and fatal edema.

Several investigators noted that the mixing of blood of certain hemophilic patients would result in the mutual correction of the clotting defect of each blood. Interpretation of these findings was eventually made by Aggeler and co-workers [*Proc. Soc. Exptl. Biol. Med.* 79:692—696 (1952)] and S. G. White et al. [*Blood* 8:101—124 (1953)]. These workers, studying a male patient with a severe hemorrahagic diathesis associated with a prolonged clotting time which was clinically indistinguishable from classic hemophilia, postulated the existence of a new clotting factor. Aggelar et al., recognizing that the new factor was a precursor of thromboplastin, named it Plasma Thromboplastin Component (PTC). The work was confirmed by Biggs et al. [*Brit. Med. J.* 2:1378—1382 (1952)] in England, who gave it the name Christmas Factor, and by Soulier and Larrieu [*New Eng. J. Med.* 249:547—553 (1953)] in France, who called it Antihemophilic Factor B. This factor is now officially designated Factor IX.

In collaboration with Aggeler et al., the first fractionated PTC preparation was prepared [*Revue d'Hematologie* 9:447—453 (1954)]. The PTC was absorbed on barium sulfate from a solution of Cohn Fraction IV and eluted with 0.34 M sodium citrate. The yields are very small, and the post-infusion in-vivo activity was only about one-fourth that predicted from in-vitro assays by a prothrombin consumption test.

Later, in collaboration with Aggeler et al., a process was developed by which PTC was adsorbed onto barium sulfate from EDTA anticoagulated plasma, eluted with a sodium chloride-sodium citrate buffer, and further purified by cold ethanol fractionation. This preparation was used clinically with excellent results [*Aggeler, P.M. et al. Trans. 6th Congress, Internat. Soc. Hematol. Grune & Stratton,* N.Y., pgs. 490—497 (1956)]. The process was later published in detail, and it was pointed out that the process was never commercialized because the Albumin and Plasma Protein Fraction obtained as by-products were contaminated with potentially hazardous levels of barium [Hink, J. H. and Johnson, F. F., The Hemophilias, CH. 18, page 156, edited by Brinkhous, Univ. of North Carolina Press (1964)]. Biggs et al. in England [*Brit. J. Haematol.* 7:349—364 (1961)] and Janiak and Soulier [*Thromb. et Diath. Haemorr.* 8:406—424 (1962)] in France later prepared Factor IX by a similar process, substituting tricalcium phosphate for the barium sulfate. However, spontaneous formation of thrombin always occurred and it was always necessary to add heparin, both during and after processing to neutralize the potentially dangerous thrombin.

Tullis et al. [*New Eng. J. Med.* 273:667—674 (1965)] have prepared and studied a somewhat similar plasma fraction, which they refer to as "prothrombin complex". In the Tullis process, the blood was collected through a calcium-removing ion exchange resin, and the resulting resin-plasma adsorbed onto DEAE cellulose. Elution was accomplished with a sodium phosphate-sodium chloride buffer stabilized with EDTA. Clinical studies on the "prothrombim complex" are described in the reference, but other data characterizing the complex are not available.

In U.S. Patent No. 3,717,708 (hereinafter referred to as '708) there is described a lyophilized mixture of coagulation components of human origin useful in the treatment of patients, who bleed due to a congential or acquired deficiency of one or more of the coagulation Factors II, VII, IX, X, prepared from human plasma collected in a citrate anticoagulant, free of heparin, free of thrombin, free of the activated form of Factor X, free of depressor activity, free of anti-complement activity, and comprising the coagulation components Factors II, VII, IX, X in essentially the same proportions as normal human plasma and of a potency equivalent to a specific activity of more than 0.5 clinical unit of each component per milligram of protein.

2

The product of '708 is produced by a process which comprises the steps of applying Cohn Supernatant I, Method 6, from unmodified citrated human plasma onto an ion exchange resin consisting essentially of cross-linked dextran chains with diethylaminoethyl groups attached by ether linkages to the glucose units of the polysaccharide chains, and adsorbing thereon said coagulation components of the plasma; selectively eluting the column with ammonium bicarbonate solution of a pH of from about 7.3 to about 8.2 and increasing molarity; separating the eluate fraction containing coagulation components; freezing the separated eluate fraction and removing the ammonium bicarbonate therefrom by lyophilizing the frozen fraction.

A method of producing a blood coagulation-promoting preparation from human blood plasma is described in U.S. Patent No. 4,160,025. The product was termed FEIBA for Factor VIII Inhibitor Bypassing Activity substance. In the production of FEIBA citrated human plasma is treated with a water-insoluble inorganic coagulation-physiologically-surface-active substance in the absence of free calcium ions to generate FEIB-activity. After separation from the above substance the supernatant is treated with an anion exchanger to adsorb FEIBA and coagulation Factors II, VII, IX, and X. The adsorbed materials are eluted from the exchanger with 3% sodium chloride (0.51 mole per liter) and 0.1% trisodium citrate dihydrate (0.0033 mole per liter). The eluate is dialyzed, frozen, and lyophilized.

A concentrate containing Factors II, IX, and X was prepared by Dike *et al.* (*British Journal of Haematology, 1972*, Vol. 22, pages 469—490). Blood plasma was diluted to one-third its volume with distilled water and adsorbed onto DEAE-cellulose in a chromatographic column. The column was washed with 0.02M sodium citrate and then eluted with an aqueous solution of 0.1M sodium chloride, 0.01M-phosphate, and 0.01M-citrate to give a Factor VII concentrate. Next, the column was eluted with 0.25 M sodium chloride, 0.01 M-phosphate, and 0.01 M-citrate to yield a concentrate containing Factors II, IX, and X.

Middleton *et al.* in *Vox Sang.*, 1973, Vol. 24, pages 441—456, described a therapeutic concentrate of coagulation Factors II, IX, and X from citrated, Factor VIII-depleted plasma. After dilution of the plasma to one-third its volume with water for injection, the plasma was contacted with a DEAE-cellulose column to adsorb the coagulation Factors. Then, the cellulose was washed with an aqueous solution containing 0.03M-phosphate and 0.03M-citrate and eluted with an aqueous solution containing 0.03M-phosphate, 0.03M-citrate, and 0.2M sodium chloride to give a concentrate of Factors II, IX, and X. This concentrate contained thrombin in amounts greater than the limits set by the U.S. National Institutes of Health. The authors note that the presence of phosphate is required in the eluting medium.

In *Vox Sang.*, 1977, Vol. 33. pages 37—50, Suomela *et al.* described a method for large-scale preparation of a coagulation Factor IX concentrate. Coagulation Factors II, VII, IX and X from human plasma are adsorbed onto DEAE Sephadex®. The gel was washed with an aqueous buffer solution containing 0.16M sodium chloride and 0.04M-phosphate and then eluted with a mixture of 0.5M and 1.0M phosphate buffer to give a Factor IX concentrate. The factor IX:Factor VII ratio of this product was about 2:1 to 3:1 and the Factor IX:NAPTT ratio was about 3:1. The thrombin activity of the above concentrate was lower than 0.001 Units per milliliter.

Summary of the invention

The present invention provides a novel method for producing blood coagulation complexes comprising coagulation Factors II, VII, IX, and X (hereinafter referred to as "Factor IX concentrates"). Surprisingly, the novel Factor IX concentrates produced by the method according to the invention possess at least about 50% more activity than the '708 products.

The method of the invention for the production of a blood plasma fraction containing coagulation Factors II, VII, IX, and X in non-activated form comprises the steps of

(a) applying a blood plasma fraction containing coagulation Factors II, VII, IX, and X to an anion exchanger to adsorb the coagulation Factors,

(b) selectively eluting the exchanger with an aqueous salt solution of increasing ionic strength and having a pH of from 6 to 9,

(c) separating the eluate fraction containing the coagulation Factors and

(d) treating the eluate to reduce the concentration of salt to a physiologically acceptable level, characterized in that in step (b) the exchanger is washed in at least two steps (b1, b2) with an aqueous solution containing a non-volatile, physiologically compatible salt and citrate ions, the ionic strength of the wash solution in step (b1) being from 0.10 to 0.45 and the ionic strength of the wash solution in step (b2) being from 0.5 to 2.5.

The combined wash step and elution step, both with an aqueous solution containing a non-volatile salt and citrate ions, may be viewed as a selective elution of the exchanger by increasing the ionic strength of the aforementioned aqueous solution in the absence of phosphate ions.

It is important that blood coagulation complexes or concentrates do not contain thrombin since the injection of thrombin into a human would be considered highly dangerous. Although it is possible to neutralize thrombin activity with heparin, it would be preferable not to have the thrombin there in the first place. Heparin is undesirable in a Factor IX containing concentrate because it also is potentially hazardous to the patient and because it causes difficulties in assaying the coagulation factors in the concentrate. In the administration of a Factor IX containing concentrate, constant monitoring of the patient's coagulation status is required, and the presence of heparin will not only complicate this testing procedure, but will make the results thus obtained unreliable. An advantage of this invention is that the concentrate be free of both thrombin and of heparin.

Another advantage of this invention is the provision of a concentrate free of the anticomplement activity which is so often associated with plasma globulins and which would make the intravenous administration of the product highly dangerous.

An important advantage of the invention is that the product thereof has substantially reduced thrombogenicity in comparison to the prior art concentrates.

A further advantage of the product of the invention is that the ratio of factor IX to Factor VII is substantially greater than that for the '708 product. Thus, the present Factor IX concentrate contains less Factor VII than does the '708 material. The present product, therefore, has a decreased ability to contribute to hemostasis.

Description of the preferred embodiments

The product of the invention is prepared from human blood plasma. The preponderance of blood generally taken and available for transfusion is protected from coagulation by treatment with a citrate anticoagulant which allows the blood to be utilized for only a limited period of time. After this limited time expires, this blood must be discarded or it can be made available for fractionation into certain useful components. As a matter of fact, the major source of whole blood converted to plasma for fractionation comes from blood collected by plasmapheresis, which has been protected by citrate anticoagulation. It is, therefore, important that processes for the fractionation of plasma, such as to obtain a Factor IX containing concentrate, be directed to citrate preserved blood, to which the instant method is.

As mentioned above, in the first step in the process of the invention a human blood plasma fraction containing coagulation Factors II, VII, IX, and X is contacted with an anion exchanger on which the coagulation Factors are adsorbed. A number of fractionation methods have been applied to human blood plasma, and these methods are summarized in "The Plasma Proteins", second edition, Vol. III, pages 548—550, Academic Press, New York, New York (1977). The preferred starting material in the process of the invention is Effluent I (Cohn Supernatant I) from a Cohn Fractionation method [U.S. Patent No. 2,390,074 and *J. Am. Chem. Soc.,* Vol. 68, page 459 (1946)]. Effluent I is contacted with an anion exchanger, for example, DEAE (diethylaminoethyl) Sephadex® consisting of cross-linked dextran chains with diethylaminoethyl groups attached by ether linkages to the glucose units of the polysaccharide chains, supplied by Pharmacia Fine Chemicals, Inc., Piscataway, New Market, N.J., to adsorb the aforementioned coagulation Factors. The spent Effluent I is returned to the plasma fractionation process so that none of the other plasma components is wasted.

Generally, contact between Effluent I and DEAE Sephadex® is achieved by forming a bed of freshly equilibrated DEAE Sephadex® and passing Effluent I therethrough. Approximately 10 g. (wet weight) of DEAE Sephadex® per liter of Effluent I is used although the amounts employed may be varied over a wide range.

The anion exchanger is washed next to remove those plasma proteins, including ceruloplasmin, that are less strongly bound to the exchanger than the coagulation Factors. The wash solution should contain a non-volatile salt and have an ionic strength sufficient to remove these less strongly bound plasma proteins but insufficient to remove the adsorbed coagulation Factors. The ionic strength (I) of the wash solution should be 0.10—0.45. A suitable non-volatile salt is sodium chloride in a concentration of 0.1—0.3 M. It is necessary that this aqueous sodium chloride solution contain citrate ions (sodium citrate or the like) at a concentration of 0.05—0.2M. A preferred wash solution in accordance with the invention is 0.2 M sodium chloride containing 0.1M sodium citrate (I=0.26). Other aqueous salt solutions may be employed with the proviso that the salt be non-volatile and physiologically compatible withthe final product. The pH of the aqueous solution is 6 to 9, preferably, 7 to 8.

Prior to this wash step it may be desired to wash the anion exchanger specifically to remove ceruloplasmin. To this end the exchanger can be washed with an aqueous solution of a volatile salt such as ammonium bicarbonate, 0.1—0.3 M. Generally the concentration of volatile salt should be sufficient to remove the ceruloplasmin but insufficient to remove the adsorbed coagulation Factors.

The anion exchanger, having been washed as described above, is treated with an aqueous solution containing a non-volatile salt and citrate ions and having an ionic strength sufficient to elute the adsorbed coagulation Factors but insufficient to remove those plasma proteins more strongly bound to the exchanger. For this purpose the ionic strength of the aqueous solution should be greater than 0.50, preferably 0.81, and within the range of 0.50—2.5. Thus, the solution should contain 0.35—2.0 M non-volatile salt and 0.005—0.02 M citrate ions. It is prefered that the eluting solution contain the same non-volatile salt as the aforementioned wash solution. A preferrred eluting solution, then is 0.35—2.0 M aqueous sodium chloride containing 0.005—0.02 M citrate ions. The pH of the eluting solution should be 6 to 9, preferably 7 to 8.

The eluate is treated to reduce its water content and the concentration of non-volatile salt, i.e., reduce the concentration of salt in the eluate, to a physiologically acceptable level, i.e., to 0.15 M or less. The aforementioned objective may be accomplished, for example, by using a combination of ultrafiltration and diafiltration as is known in the art. It is preferred that the ultrafiltration and diafiltration be carried out against an aqueous sodium chloride-sodium citrate buffer in final container concentration, i.e., the concentration of the above as found in the final container. Generally, final container concentration of sodium chloride is 0.088 M and of sodium citrate, 0.05 M. Other means of achieving removal of the salt will be suggested to those skilled in the art. It is to be noted that reduction of the amount of water may be accomplished by other methods with the exception of lyophilization, which is to be avoided.

The Factor IX concentrates can be formulated into pharmaceutical preparations for therapeutic, diagnostic, or other uses. To prepare them for intravenous administration the compositions are dissolved usually in water containing physiologically compatible substances such as sodium chloride and glycine, and having a buffered pH compatible with physiological conditions. Generally, guidelines for intravenously administered compositions are established by governmental regulations.

The product prepared in accordance with the above method is characterized as substantially free of thrombogenicity and free of thrombin, heparin, thromboplastin activity, anticomplement activity, depressor activity, and activated Factor X, comprising coagulation Factors II, VII, IX and X. It has a specific activity of at least about 1.5 Factor IX units per mg. total protein. One Factor IX unit is defined as that amount of Factor IX activity contained in one ml. of fresh average pooled plasma. The Factor IX:Factor VII ratio is at least 6:1 or more, preferably 9:1 or more. The Factor IX:Factor II ratio and the Factor IX:Factor X ratio are 1:1 to 2:1. Factor IX:NAPTT is 5:1 or greater.

A modification of the product of this invention is the product in an electrolyte buffer suitable for intravenous administration as follows:

The ultrafiltered/diafiltered eluate is sterilized by filtration through a sterile 0.2 micron porosity membrane filter and aseptically filled in convenient portions into sterile bottles, frozen and lyophilized and stored at 5°C.

For human administration, the content of the vial is aseptically redissolved in water for injection. Alternatively, the product can be sterile filled and lyophilized to a water-free product. In this instance it is reconstituted with a suitable isotonic diluent prior to human injection.

The above process recites the details which are preferred at this time. However, many variations can be made without departing from the general principles of the invention.

For example, the amount of DEAE Sephadex® relative to the volume of Effluent I may be varied over a wide range. The use of larger amounts of DEAE Sephadex® will result in a lower degree of purity and greater yield of the coagulation complex, and a greater loss of other plasma proteins normally recoverable in subsequent steps. The use of smaller amounts of DEAE Sephadex® will result in even higher degrees of purity, but will also result in considerably lower yields. Although DEAE Sephadex® is the preferred anionic exchanger, presumably other anionic adsorbents would work also.

The elution step with an aqueous solution containing a non-volatile salt and citrate ions is extremely important to the process of this invention. Only in this fashion is there obtained a product having at least about 50% more activity than the '708 product. Furthermore, it is only in this manner that a less thrombogenic product can be secured.

Examples

The invention is demonstrated further by the following illustrative examples.

Assay methods
Factors II and VII

Factor II and Factor VII were assayed by the method of Owren described in the *Scand. J. Clin. and Lab. Investigation*, Vol. 1, page 81 (1949).

Factors X and Xa

Factor X and Factor Xa were assayed by the method of Bachmen *et al*, described in *Thromb. Diath. Haemorrh.*, Vol. 2, page 24, (1958).

Thrombin

The assay procedure employed was described by Fenton II *et al,* in *Thrombosis Res.*, Vol. 4, pages 809—817 (1974).

Factors IX and VIII

Modification of the procedures described by Langdell *et al* (partial thromboplastin time technique), *J. Lab. Clin. Med.,* Vol. 41, pages 637—647 (1953) and Proctor *et al* (kaolin clotting time method) *Amer. J. Clin. Path.,* Vol. 36, page 212 (1961) were employed. Platelet Factor 3 was supplied by a cephalin suspension. Maximum surface contact activation was achieved with Celite® powder. All other clotting factors (except Factor IX or Factor VIII) were supplied by a substrate comprising plasma from a patient severely deficient in Factor IX or Factor VIII mixed with barium sulfate adsorbed beef plasma. Quantitation of an unknown specimen was made by comparing its clotting time in the test with that achieved by dilutions of a normal standard.

The exact assay procedure is the same for both Factor IX and Factor VIII except that the activator in Factor IX assay is Plateline® Plus Activator instead of automated APPT reagent (General Diagnostics, Inc., Morris Plains, New Jersey).

Non-activated partial thromboplastin time (NAPTT)

A 0.1-ml. sample of NAPTT Substrate Plasma (stored on ice), a 0.1-ml. sample of Partial Thromboplastin with no activator (stored on ice), and 0.1-ml. of the sample to be tested (in various dilutions) were added to a 10×75 mm polystyrene test tube, mixed by shaking carefully and placed in 37°C water bath with simultaneous starting of a stopwatch. After exactly a one-minute period, 0.1 ml 0.025 M $CaCl_2$ maintained at 37°C was added with simultaneous starting of the timer and the contents of the tube were mixed by careful shaking. Thereafter, the tube was held undisturbed for 30 seconds to 1 minute, depending upon the type of sample. It was then tilted from time to time until gelation started or a clot was observed with care being taken to minimize exposure to room temperature which was below 37°C. Time was recorded at the first sign of a clot formation. Development of opacity and gelation preceded the formation of a firm clot.

When the sample was replaced by Tris buffer, NAPTT blank time was obtained. If the blank time was over 300 seconds, the assay was continued. Any substrate plasma which gave a blank time of less than 300 seconds was unacceptable for this test.

By obtaining NAPTT times at a wide range of dilutions and plotting times in seconds on ordinate and dilutions on abscissa, a straight line relation was obtained. In most prothrombin complex concentrates, inhibitory effect was observed at low dilutions. Therefore, a straight line relationship was observed at 1:100 and higher dilutions. By using a suitable standard such as Factor IX-1 (FN-1) of the Bureau of Biologics and assigning 100 units per ml for this standard, it was possible to construct a standard curve. On this standard curve, the NAPTT times of a given sample were read and expressed as units. Longer NAPTT times (close to blank time) expressed as low NAPTT units/ml indicate reduced thrombogenicity.

Example 1

Effluent I (1500 liters) was contacted with 15 kg. of DEAE Sephadex® gel and mixed together. After two hours the mixture was filtered to give 15 kg. of gel which was washed sequentially with 50 liters of 0.2 M ammonium bicarbonate, 50 liters of 0.3 M ammonium bicarbonate and 45 liters of 0.01 M sodium citrate-0.2 M sodium chloride (ph 7.1) buffer. Elution was carried out with 33.7 liters of 0.01 M sodium citrate-0.75 M sodium chloride at pH 7.24 to obtain a protein solution of $A_{280}$=10.2. This was concentrated by ultrafiltration to $A_{280}$ of about 38.2 and diafiltered to 5 volume exchange against 0.05 M sodium citrate-0.088 M sodium chloride (pH 7.19) buffer.

The above material was sterile filtered, sterile filled in vials (20 ml. fill volume) and freeze dried. The contents of the vials were assayed by the aforementioned methods. The results are summarized in Table 1.

TABLE 1

| Coagulation factors (U/ml) | | | | Specific activity $(U/mg)^a$ | NAPTT | |
|---|---|---|---|---|---|---|
| II | VII | IX | X | | $(sec)^b$ | (U/ml) |
| 29 | 4.3 | 51.7 | 36 | 2.21 | 248 | 7.2 |

[a]Units IX per mg. of total protein
[b]1:100 dilution

Example 2

Effluent I (1300 liters) was contacted with 13 kg. of DEAE Sephadex® gel and mixed together. After two hours the mixture was filtered to give 13 kg. of gel which was washed sequentially with 50 liters of 0.2 M ammonium bicarbonate, 55 liters of 0.3 M ammonium bicarbonate and 45 liters of 0.01 M sodium citrate-0.2 M sodium chloride (pH 7.18) buffer. Elution was carried out with 36.5 liters of 0.01 M sodium citrate-0.75 M sodium chloride (pH 7.16) to obtain a protein solution of $A_{280}$=9.65. This was concentrated by ultrafiltration to $A_{280}$=29.3 and diafiltered to 5 volume replacement against 0.05 M sodium citrate-0.088 M sodium chloride (pH 7.16).

The above material was sterile filtered, sterile filled (20 ml. fill volume) and freeze dried. The contents of the vials were assayed by the aforementioned methods. The results are outlined in Table 2.

TABLE 2

| Coagulation factors (U/ml) | | | | Specific activity (U/mg) | NAPTT | |
|---|---|---|---|---|---|---|
| II | VII | IX | X | | $(sec)^a$ | (U/ml) |
| 24 | 5.4 | 37 | 39 | 1.53 | 272 | 5.8 |

[a]1:100 dilution

# 0 041 174

Example 3

Table 3 contains a side-by-side comparison of the products of Examples 1 and 2 and products prepared by the process of '708 (as described at columns 3 and 4).

TABLE 3

| Product | IX (U/ml) | Specific activity (IX/mg protein) | NAPTT (U/ml) | IX/NAPTT |
|---|---|---|---|---|
| Invention[a] | 44.4 | 1.9 | 6.8 | 6.54 |
| '708[b] | 27.1 | 1.1 | 8.9 | 3.30 |

[a]Average of 2 runs
[b]Average of 5 runs

Example 4

A comparison of the concentrations of the coagulation Factors in the product of the invention prepared in Examples 1 and 2 and the product of '708 is outlined in Table 4.

TABLE 4
Coagulation factors (U/ml)

| Product | II | VII | IX | X | IX/VII |
|---|---|---|---|---|---|
| Invention[a] | 26.5 | 4.9 | 44.4 | 37.5 | 9.44 |
| '708[a] | 18.3 | 9.5 | 26.1 | 29.3 | 2.80 |

[a]Average of 2 runs

Example 5

The procedure of Example 2 was employed to prepare a product in accordance with the invention. The so-prepared product was assayed by the aforementioned methods and the held for a period of 8 months to determine its *in vitro* stability. The so-held product was assayed as above and the results are outlined in Table 5.

TABLE 5

| Product held | Coagulation factors (U/ml) | | | NAPTT 1:10[a] (sec.) | 1:100[a] (sec.) |
|---|---|---|---|---|---|
| | II | IX | X | | |
| 0 months | 24.2 | 38.7 | 37.5 | 161 | 259 |
| 8 months | 21.8 | 40.2 | 40.8 | 172 | 277 |

[a]Dilution

**Claims**

1. A process for the production of a blood coagulation complex containing coagulation Factors II, VII, IX, and X in non-activated form, which comprises the steps of

(a) applying a blood plasma fraction containing coagulation Factors II, VII, IX, and X to an anion exchanger to adsorb the coagulation Factors,

(b) selectively eluting the exchanger with an aqueous salt solution of increasing ionic strength and having a pH of from 6 to 9,

(c) separating the eluate fraction containing the coagulation Factors and

(d) treating the eluate to reduce the concentration of salt to a physiologically acceptable level, characterized in that in step (b) the exchanger is washed in at least two steps (b1, b2) with an aqueous solution containing a non-volatile, physiologically compatible salt and citrate ions, the ionic strength of the wash solution in step (b1) being from 0.10 to 0.45 and the ionic strength of the wash solution in step (b2) being from 0.5 to 2.5.

7

2. A process according to claim 1, characterized in that the wash solution in step (b1) comprises 0.1 to 0.3 M sodium chloride and 0.05 to 0.2 M citrate ions.

3. A process according to claim 1 or 2, characterized in that the wash solution in step (b2) comprises 0.35 to 2.0 M sodium chloride and 0.005 to 0.02 M citrate ions.

4. A process according to any of claims 1 to 3, characterized in that prior to step (b) the exchanger is washed with a 0.1 to 0.3 M aqueous solution of a volatile salt.

5. A process according to claim 4, characterized in that the volatile salt is ammonium bicarbonate.

6. A blood coagulation complex containing coagulation Factors II, VII, IX, and X in non-activated form capable of being prepared by the process of any of claims 1 to 5.

7. A lyophilized stable composition for controlling bleeding in hemophilia containing coagulation Factors II, VII, IX and X in non-activated form prepared by a process which comprises the steps of

(a) applying a blood plasma fraction containing coagulation Factors II, VII, IX, and X to an anion exchanger to adsorb the coagulation Factors,

(b) selectively eluting the exchanger with an aqueous salt solution of increasing ionic strength and having a pH of from 6 to 9,

(c) separating the eluate fraction containing the coagulation Factors,

(d) treating the eluate to reduce the salt concentration to a physiologically acceptable level and, optionally, subjecting the eluate to sterile filtration, and

(e) lyophilizing the eluate, characterized in that in step (b) the exchanger is washed in at least two steps with an aqueous solution containing a non-volatile, physilogically compatible and citrate ions.

8. A sterile lyophilized storage stable concentrate for controlling bleeding in hemophilia, free of thrombin, heparin, thromboplastin activity, anticomplement activity, depressor activity, and activated Factor X, and containing coagulation Factors II, VII, IX, and X, in non-activated form, having a specific activity of at least 1.5 Factor IX units per milligram of total protein and a Factor IX:Factor VII ratio of at least 6:1.

9. The concentrate of claim 8 wherein the Factor IX: NAPTT ratio is at least 5:1 on a Units per milliliter basis.

**Revendications**

1. Un procédé pour la production d'un complexe de coagulation du sang contenant les facteurs de coagulation II, VII, IX et X sous une forme non activée, qui comprend les stades de

(a) application d'une fraction de plasma sanguin contenant les facteurs de coagulation II, VII, IX et X à un échangeur d'anions pour adsorber les facteurs de coagulation,

(b) élution sélective de l'échangeur avec une solution aqueuse de sel de force ionique croissante et ayant un pH de 6 à 9,

(c) séparation de la fraction d'éluat contenant les facteurs de coagulation, et

(d) traitement de l'éluat pour réduire la concentration du sel à une valeur physiologiquement acceptable,

caractérisé en ce que dans le stade (b), l'échangeur est lavé en au moins deux stades (b1, b2) avec une solution aqueuse contenant un sel non volatil physiologiquement compatible et des ions citrates, la force ionique de la solution de lavage dans le stade (b1) étant de 0,10 à 0,45, et la force ionique de la solution de lavage dans le stade (b2) étant de 0,5 à 2,5.

2. Un procédé selon la revendication 1, caractérisé en ce que la solution de lavage dans le stade (b1) comprend du chlorure de sodium 0,1 à 0,3 M et des ions citrates de 0,05 à 0,2 M.

3. Un procédé selon la revendication 1 ou 2, caractérisé en ce que la solution de lavage dans le stade (b2) comprend du chlorure de sodium 0,35 à 2,0 M et des ions citrates à 0,005 à 0,02 M.

4. Un procédé selon l'une quelconque des revendications 1 à 3, caractérisé en ce qu'avant le stade (b) on lave l'échangeur avec une solution aqueuse 0,1 à 0,3 M d'un sel volatil.

5. Un procédé selon la revendication 4, caractérisé en ce que le sel volatil est le bicarbonate d'ammonium.

6. Un complexe de coagulation du sang contenant les facteurs de coagulation II, VII, IX et X sous une forme non activée pouvant être préparé selon le procédé de l'une quelconque des revendications 1 à 5.

7. Une composition stable lyophilisée pour lutter contre l'hémorragie dans l'hémophilie, contenant les facteurs II, VII, IX et X sous une forme non activée préparée selon un procédé qui comprend les stades de:

(a) application d'une fraction de plasma sanguin contenant les facteurs de coagulation II, VII, IX et X à un échangeur d'anions pour adsorber les facteurs de coagulation,

(b) élution sélective de l'échangeur avec une solution aqueuse de sel de force ionique croissante et ayant un pH de 6 à 9,

(c) séparation de la fraction d'éluat contenant les facteurs de coagulation,

(d) traitement de l'éluat pour réduire sa concentration en sel à une valeur physiologiquement acceptable et, éventuellement, filtration stérile de l'éluat, et

(e) lyophilisation de l'éluat, caractérisée en ce que dans le stade (b), on lave l'échangeur en au moins deux stades avec une solution aqueuse contenant un sel non volatil physiologiquement compatible et des ions citrates.

8. Un concentré stérile lyophilisé stable au stockage pour lutter contre l'hémorragie dans l'hémophilie, dépourvu de thrombine, d'héparine, d'activité thromboplastinique, d'activité anti-complémentaire, d'activité dépressive et du facteur IX activé et contenant les facteurs de coagulation II, VII, IX et X sous une forme non activée, ayant une activité spécifique d'au moins 1,5 unité de facteur IX par milligramme de protéines totales et un rapport facteur IX/facteur VII d'au moins 6/1.

9. Le concentré de la revendication 8, dans lequel le rapport facteur IX/NAPTT est d'au moins 5/1, les valeurs étant exprimées en unités par millilitre.

**Patentansprüche**

1. Verfahren zur Herstellung eines Blutgerinnungs-Komplexes, der die Gerinnungsfaktoren II, VII, IX und X in nicht-aktivierter Form enthält, welches die folgenden Schritte umfasst:

(a) Aufbringen einer Blutplasmafraktion, welche die Gerinnungsfaktoren II, VII, IX und X enthält, auf einen Anionenaustauscher, um die Gerinnungsfaktoren zu adsorbieren,

(b) selektives Eluieren des Austauschers mit einer wässrigen Salzlösung von zunehmender Ionenstärke und einem pH von 6 bis 9,

(c) Abtrennen der Eluatfraktion, welche die Gerinnungsfaktoren enthält, und

(d) Behandeln des Eluats zur Reduzierung der Salzkonzentration auf einen physiologisch annehmbaren Gehalt, dadurch gekennzeichnet, dass in Schritt (b) der Austauscher in mindestens zwei Schritten (b1, b2) mit einer wässrigen Lösung gewaschen wird, die ein nicht-flüchtiges, physiologisch kompatibles Salz und Citrationen enthält, wobei die Ionenstärke der Waschlösung in Schritt (b1) 0,10 bis 0,45 und die Ionenstärke der Waschlösung in Schritt (b2) 0,5 bis 2,5 beträgt.

2. Verfahren nach Anspruch 1, dadurch gekennzeichnet, dass die Waschlösung in Schritt (b1) 0,1 bis 0,3 M Natriumchlorid und 0,05 bis 0,2 M Citrationen umfasst.

3. Verfahren nach Anspruch 1 oder 2, dadurch gekennzeichnet, dass die Waschlösung in Schritt (b2) 0,35 bis 2,0 M Natriumchlorid und 0,005 bis 0,02 M Citrationen umfasst.

4. Verfahren nach einem der mehreren der Ansprüche 1 bis 3, dadurch gekennzeichnet, dass vor dem Schritt (b) der Austauscher mit 0,1 bis 0,3 M wässriger Lösung eins flüchtigen Salzes gewaschen wird.

5. Verfahren nach Anspruch 4, dadurch gekennzeichnet, dass das flüchtige Salz Ammoniumbicarbonat darstellt.

6. Blutgerinnungs-Komplex, der die Gerinnungsfaktoren II, VII, IX und X in nicht-aktivierter Form enthält und nach dem Verfahren von einem oder mehreren der Ansprüche 1 bis 5 hergestellt werden kann.

7. Lyophilisierte stabile Zubereitung zur Steuerung der Blutung bei Hemophilie, welche die Gerinnungsfaktoren II, VII, IX und X in nicht-aktivierter Form enthält, hergestellt nach einem Verfahren, das die folgenden Schritte umfasst:

(a) Aufbringen einer Blutplasmafraktion, welche die Gerinnungsfaktoren II, VII, IX und X enthält, auf einen Anionenaustauscher, um die Gerinnungsfaktoren zu adsorbieren,

(b) selektives Eluieren des Austauschers mit einer wässrigen Salzlösung von zunehmender Ionenstärke und einem pH von 6 bis 9,

(c) Abtrennen der Eluatfraktion, welche die Gerinnungsfaktoren enthält, und

(d) Behandeln des Eluates zur Reduzierung der Salzkonzentration auf einen physiologisch annehmbaren Gehalt und, sofern dies gewünscht wird, Unterziehen des Eluates einer Sterilfiltration, und

(e) Lyophilsieren des Eluates, dadurch gekennzeichnet, dass in Schritt (b) der Austauscher mindestens in zwei Schritten mit einer wässrigen Lösung gewaschen wird, welche ein nicht-flüchtiges, physiologisch kompatibles Salz und Citrationen enthält.

8. Steriles, lyophilisiertes, lagerstabiles Konzentrat zur Steuerung der Blutung bei Hemophilie, das frei ist von Thrombin, Heparin, Thromboplastin-Aktivität, Antikomplement-Aktivität, Depressor-Aktivität und aktiviertem Faktor X, und die Gerinnungsfaktoren II, VII, IX und X in nicht-aktivierter Form enthält, mit einer spezifischen Aktivität von mindestens 1,5 Faktor IX-Einheiten pro mg des Gesamtproteins und einem Faktor IX:Factor VII-Verhältnis von mindestens 6:1.

9. Konzentrat nach Anspruch 8, dadurch gekennzeichnet, dass das Faktor IX:NAPTT-Verhältnis mindestens 5:1 auf Einheiten-pro-ml-Basis beträgt.